Europäisches Patentamt

European Patent Office

Office européen des brevets

(19)

(11) Veröffentlichungsnummer : **0 461 431 B1**

(12) **EUROPÄISCHE PATENTSCHRIFT**

(45) Veröffentlichungstag der Patentschrift :
**16.03.94 Patentblatt 94/11**

(51) Int. Cl.⁵ : **C07C 19/045,** C07C 17/02,
B01J 35/02

(21) Anmeldenummer : **91108296.4**

(22) Anmeldetag : **23.05.91**

(54) **Zylindrisch geformter Katalysator und dessen Verwendung bei der Oxichlorierung von Ethylen.**

(30) Priorität : **09.06.90 DE 4018512**

(43) Veröffentlichungstag der Anmeldung :
**18.12.91 Patentblatt 91/51**

(45) Bekanntmachung des Hinweises auf die
Patenterteilung :
**16.03.94 Patentblatt 94/11**

(84) Benannte Vertragsstaaten :
**BE DE FR GB IT NL SE**

(56) Entgegenhaltungen :
**EP-A- 0 102 641**
**DE-A- 3 113 179**
**DE-A- 3 607 449**
**US-A- 4 717 781**

(73) Patentinhaber : **Degussa Aktiengesellschaft**
**Weissfrauenstrasse 9**
**D-60311 Frankfurt (DE)**
(84) **BE DE FR GB IT NL SE**
Patentinhaber : **WACKER-CHEMIE GMBH**
**Hanns-Seidel-Platz 4**
**D-81737 München (DE)**
(84) **DE**

(72) Erfinder : **Deller, Klaus, Dr.**
**Friedhofstrasse 47**
**W-6452 Hainburg (DE)**
Erfinder : **Krause, Helmfried**
**Odenwaldstrasse 39**
**W-6458 Rodenbach 1 (DE)**
Erfinder : **Schmidhammer, Ludwig, Dr.**
**Pappelweg 5**
**W-8261 Haiming (DE)**
Erfinder : **Dafinger, Willibald, Dr.**
**Goethestrasse 22**
**W-8261 Emmerting (DE)**

## Beschreibung

Die Erfindung betrifft einen geformten Trägerkatalysator, der Kupferionen und Alkaliionen auf einem zylindrisch geformten, aktivierten Träger enthält. Dieser Trägerkatalysator dient insbesondere zur Verwendung bei der selektiven Oxichlorierung von Ethylen.

Die Oxichlorierung von Ethylen in einem Katalysatorfestbett ist ein bekanntes, großtechnisch ausgeübtes Verfahren: Die dabei verwendeten Katalysatoren enthalten auf einem Träger Kupfer-II-Chlorid in Verbindung mit Promotoren wie z. B. Kaliumchlorid. Bei der Durchführung des Verfahrens ist es besonders erwünscht, daß der durch den Katalysator verursachte Druckverlust gering, die wirksame Oberfläche des Katalysators groß und die Wärmeleitfähigkeit zwischen den Katalysatorteilchen und einem gegebenenfalls vorgesehenen inerten Verdünnungsmittel gut ist.

Bei der Verwendung geformter Trägerkatalysatoren ist der Druckverlust der Katalysatorschüttung in der Regel sehr groß, da die Hohlräume in einem dichtgepackten Bett klein sind. Bezüglich der Formgestaltung des Trägermaterials, das üblicherweise aus aktivierter Tonerde, Aluminiumsilikat, Silicagel oder ähnlichem oberflächenaktiven Material besteht, sind beispielsweise zylindrische Formkörper (Hohlzylinder) in der DE-OS 36 07 449 (= EP-A 240 714) und in der DE-OS 31 13 179 (= US-PS 4 366 093) bzw. Kugeln in der DE-OS 26 30 938 beschrieben. Die Erniedrigung des Druckverlustes, z. B. durch Vergrößern der Maße für Durchmesser und Länge der Formlinge führt jedoch meist nur zu kleineren Umsätzen, da die wirksame Oberfläche des Katalysators dadurch reduziert wird.

Bei der Oxichlorierungsreaktion wird viel Wärme frei, wodurch trotz Kühlung der Rohrbündelreaktoren durch Verdampfen von heißem Druckwasser oder mit Hilfe eines Wärmeträgermediums (z. B. Thermoöl oder eine Salzschmelze) im Katalysatorbett weit überhitzte Zonen (Hot Spots) entstehen, die den Katalysator schädigen und die Selektivität reduzieren. Es wurde deshalb gemäß DE-OS 14 93 213 bereits vorgeschlagen, die Oxichlorierung in einem System mehrerer in Reihe geschalteter Reaktoren durchzuführen und die Gesamtmenge des für die Oxichlorierungsreaktion benötigten Sauerstoffs auf die entsprechende Anzahl der Reaktoren aufzuteilen sowie die Reaktoren mit Katalysatoren unterschiedlicher, in Strömungsrichtung zunehmender Aktivitätsstufen zu befüllen. Es wurde ferner vorgesehen, den Oxichlorierungskatalysator durch Zuschlag von Inertmaterial abzumagern, um Überhitzungen im Katalysatorbett zu vermeiden. Ein technisch wie wirtschaftlich aufwendiges Verfahren beschreibt die US-PS 3 892 816, wonach die Reaktion in Gegenwart eines hohen Ethylenüberschusses mit reinem Sauerstoff unter Kreislaufführung des überschüssigen, nicht umgesetzten Ethylens durchgeführt wird.

In all diesen Fällen erfolgt die Reaktionskontrolle mithin auf Kosten der Raum-Zeit-Leistung einer gegebenen Anlage. Wegen der stark exothermen Reaktion ist es auch wichtig, daß im Katalysatorbett eine gute Wärmeleitfähigkeit herrscht, was meist dadurch erreicht wird, daß man eine Katalysatorform wählt, bei der die Kontaktflächen zwischen den einzelnen Katalysatorteilchen möglichst groß ist, z. B. die Ringform, die beispielsweise durch Extrudieren oder Tablettieren zugänglich ist, oder die preiswertere Form säulenartiger Träger. Das sich als Zielkonflikt darstellende Problem, bei gegebener Anlage ohne Einbuße der Raum-Zeit-Leistung, die u. a. auch vom Druckabfall über die Reaktoren und die Katalysatoraktivität abhängig ist, hohe Selektivitäten bei niedrigen Verbrennungsraten zu erzielen, wird durch die bekannten Verfahren nicht in wirtschaftlicher Weise gelöst, zumal das üblicherweise verwendete Trägermaterial, dessen Porenverteilung gewöhnlich ein Porenmaximum von 3 bis 10 Nanometer aufweist, erfahrungsgemäß die Oxidation von Ethylen zu unerwünschtem Kohlenmonoxid und Kohlendioxid stark fördert.

Der Erfindung liegt die Aufgabe zugrunde, einen Katalysator der eingangs genannten Art zur Verfügung zu stellen, der im Vergleich zu den Katalysatoren des Standes der Technik unter Wahrung wirtschaftlicher Aspekte hinsichtlich Druckabfall, Aktivität und Selektivität überlegene Eigenschaften aufweist.

Diese Aufgabe wird gelöst mit einem zylindrisch geformten Trägerkatalysator, enthaltend Kupferionen und Alkaliionen auf einem ringförmigen Träger, der dadurch gekennzeichnet ist, daß das ringförmige Trägermaterial einen Außendurchmesser von 4 bis 6 mm, einen Innendurchmesser von 1 bis 2 mm und eine Höhe aufweist, die das 1,7 bis 3,75 fache, vorzugsweise das 2,0 bis 3,00 fache des Außendurchmessers ausmacht, der die Kupferionen und Alkaliionen enthaltende Trägerkatalysator ein Gesamtporenvolumen von 0,6 bis 1,0 ml/g hat und die Porenverteilung sich so darstellt, daß Poren kleiner 4 Nanometer nicht vorhanden sind, mindestens 80 % des Gesamtporenvolumens durch Poren mit einem Durchmesser von 8 bis 20 Nanometer gebildet werden und der Rest aus Makroporen größer 20 Nanometer besteht.

Besonders vorteilhaft ist, wenn der Träger aus Gamma-Aluminiumoxid mit einer spezifischen Oberfläche von 120 bis 215 m$^2$/g (BET-Oberfläche) und einem Porenvolumen von 0,6 bis 1,0 ml/g besteht.

Ein Gegenstand der Erfindung ist auch die Verwendung des Trägerkatalysators zur Herstellung von 1,2-Dichlorethan durch Oxichlorierung von Ethylen mit Luft oder mit Sauerstoff angereicherter Luft oder mit reinem Sauerstoff unter Kreisgasführung des überschüssigen, nicht umgesetzten Ethylens.

EP 0 461 431 B1

Es ist zwar allgemein bekannt, daß man den Druckabfall über ein Bett aus ring- oder kugelförmigen Katalysatorkörpern, das bezüglich Aktivität und Druckverlust im Vergleich zu Betten mit anderen Geometrien von Katalysatorkörpern besonders vorteilhaft ist, durch Anwendung größerer Außendurchmesser und/oder Längen der Katalysatorkörper reduzieren kann.

Überraschenderweise wird jedoch damit trotz Erniedrigung der wirksamen Oberfläche die Aktivität bzw. der Umsatz erhöht, wenn das erfindungsgemäße Trägermaterial verwendet wird.

Der erfindungsgemäße, ringförmige, Kupfer- und Alkaliionen enthaltende Trägerkatalysator mit der speziellen Gestalt, dem beschriebenen Porenvolumen und der beschriebenen Porenverteilung weist also einen geringeren Druckverlust als herkömmliche Hohlextrudate auf und ist trotz der verkleinerten wirksamen Oberfläche aktiver.

Niedrigere Druckverluste erhöhen auch die Raum-Zeit-Ausbeuten, weil bestehende Anlagen dadurch höher belastbar sind. Daneben erzielt man mit dem erfindungsgemäßen Trägerkatalysator auch höhere Selektivitäten und reduziert dabei besonders die Oxidation von Ethylen zu Kohlenmonoxid und/oder Kohlendioxid, wodurch die Ethylenausbeute verbessert wird. Dies ist umso überraschender, weil durch die erfindungsgemäße spezielle Gestalt und Abmessung des Trägermaterials die Wärmeleitfähigkeit zwischen den Katalysatorteilchen an sich schlechter sein sollte. Daneben war für den Fachmann nicht ohne weiteres erkennbar bzw. vorhersehbar, daß bei Übergang auf Trägermaterialien mit relativ großem Gesamtporenvolumen, wobei aber der überwiegende Teil der Poren im Mesoporenbereich liegt, trotz Verkleinerung der wirksamen Oberfläche des Katalysators eine Aktivitätssteigerung erfolgt. Normalerweise ist nämlich die Aktivität eines Katalysators eine Funktion der Porenverteilung, wobei mit innerhalb bestimmter Grenzen abnehmendem Porendurchmesser die Aktivität ansteigt.

Aufgrund des großen Porenvolumens sind die erfindungsgemäßen Trägerkatalysatoren auch sehr langlebig und liefern bei großen Raum-Zeit-Leistungen eine extrem hohe spezifische Produktausbringung. Die spezifische Oberfläche wird nach BET gemäß DIN 66131 bestimmt. Das Gesamtporenvolumen errechnet sich aus der Summe der Mikro-, Meso- und Makroporenvolumina, wobei die Mikro- und Mesoporenvolumina durch Aufnahme von Stickstoffisothermen bzw. deren Auswertung nach BET, de Boer, Barret, Joyner und Halenda gemessen werden, während die Makroporenvolumina durch das Quecksilbereinpreßverfahren ermittelt werden.

Die Verformung des Trägermaterials kann auf übliche Weise unter Benutzung der bekannten Verfahren wie Tablettierung oder vorzugsweise Hohlextrudierung erfolgen, wobei in der Regel die bekannten Extrudierhilfsmittel wie Stärke, Methylcellulose, Polyethylenglykol und dergleichen als Zusatz mitverwendet werden. Diese Mittel wirken porositätsverbessernd und/oder als Gleitmittel und/oder als Peptisierungsmittel und werden mit dem Trägermaterial, bevorzugt erst nach einem Knetprozeß, verformt. Nach der Formgebung und der erforderlichen Wärmebehandlung, die in einem oder aber auch in mehreren Calcinierschritten erfolgen kann, wird der Träger mit den Aktivkomponenten bzw. Promotoren getränkt. Das Imprägnieren des Trägers mit den katalytisch wirksamen Komponenten geschieht dabei nach den üblichen Techniken, z. B. durch Tränken mit wäßrigen Lösungen, in denen Kupfer-II-Chlorid und Alkalichlorid, meist Kaliumchlorid, in den gewünschten Mengen vorhanden sind.

Die Trocknung des Trägerkatalysators wird in Luft oder Inertgasatmosphäre bei Temperaturen von 80 bis 180° C vorgenommen.

Daneben kann aber der Verformungsschritt auch von einem bereits die Aktivkomponenten enthaltenden pulverförmigen Trägerkatalysator ausgehen. Als Trägermaterial wird bevorzugt Gamma-Aluminiumoxid mit einer spezifischen Oberfläche von 120 - 215 m²/g und einem Porenvolumen von 0,6 - 1,0 ml/g verwendet, um nach dem Imprägnieren des Trägers mit den Aktivkomponenten den erfindungsgemäßen Bereich der spezifischen Oberfläche und des beanspruchten Porenvolumens und gegebenenfalls eines fertigen Katalysators mit Aluminiumoxidträger einhalten zu können.

Die Durchführung des Oxichlorierungsverfahrens unter Verwendung des erfindungsgemäßen Katalysators kann ein- oder auch mehrstufig erfolgen. Bei der bevorzugten mehrstufigen Reaktionsführung werden die einzelnen Einsatzstoffe wie Luft bzw. Sauerstoff oder Chlorwasserstoff aufgeteilt und den einzelnen Stufen getrennt zugeführt.

Das aus der Reaktionszone austretende Reaktionsgemisch kann bei einstufiger und bei mehrstufiger Fahrweise mit frischem Chlorwasserstoff, Ethylen und Luft bzw. Sauerstoff vermischt in die Reaktionszone zurückgeführt werden, wobei gegebenenfalls die Reaktionsprodukte 1,2-Dichlorethan und Wasser vor der Rückführung vollständig oder zum Teil aus dem Reaktionsgemisch abgetrennt werden.

Als Sauerstoffquelle dient Luft bzw. mit Sauerstoff angereicherte Luft oder auch reiner Sauerstoff, wobei sauerstoffreicher Luft in einer günstigen Ausführungsform noch Wasserdampf zugegeben wird.

Bei Verwendung reinen Sauerstoffs ist ein großer Ethylenüberschuß erforderlich und das überschüssige, nicht umgesetzte Ethylen muß im Kreis gefahren werden. Dabei werden die Vorteile des erfindungsgemäßen Trägerkatalysators in keiner Weise abgeschwächt. Die Temperaturen in den Katalysatorschüttungen bewegen

3

sich üblicherweise bei Werten zwischen 200 und 320° C und die Drücke bei 3 bis 10 bar absolut. Um zu hohe Temperaturspitzen im Katalysatorbett zu vermeiden, ist es bei der Durchführung des Oxichlorierungsverfahrens unter Verwendung des erfindungsgemäßen Trägerkatalysators zweckmäßig, die Aktivität des Katalysators abzustufen, so daß die Aktivität im Reaktor bei einstufiger Fahrweise bzw. bei mehrstufiger Durchführung zumindest in der ersten und zweiten Stufe in Produktstromrichtung zunimmt. Die Abstufung der Katalysatoraktivität kann durch an sich bekannte Maßnahmen erfolgen, z. B. durch Zugabe von Inertmaterial als Verdünnungsmittel. Bevorzugt wird jedoch die jeweils gewünschte Katalysatoraktivität durch entsprechende Änderung der Kupfer-II-Chlorid-Konzentration des Katalysators und/oder des Molverhältnisses von Kupfer-II-Chlorid zu Alkalichlorid im Katalysator eingestellt.

Die Erfindung wird durch die nachstehend beschriebenen Beispiele weiter erläutert.

Beispiel 1

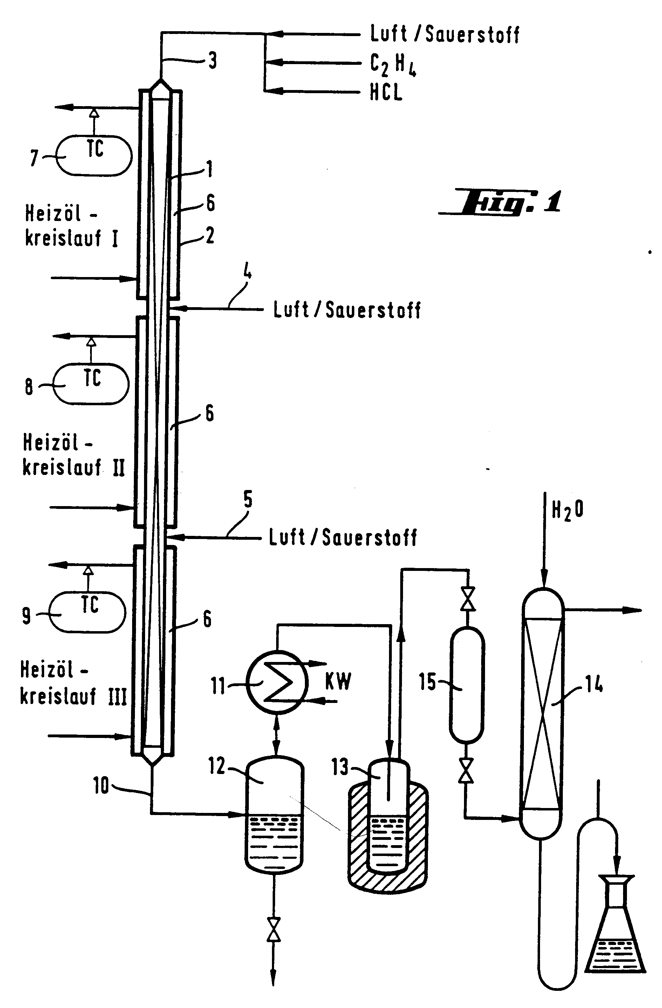

Der verwendete Laborreaktor besteht gemäß Figur 1 aus einem senkrecht stehenden Nickelrohr (1) mit 25 mm lichter Weite und 2000 mm Länge und ist von einem Doppelmantel (2) aus Stahl umgeben. Der Reaktor verfügt über drei Zuleitungen, wobei die Zuleitstelle (3) am oberen Ende des Reaktionsrohres angeordnet ist, während die Zuleitstellen (4) und (5) nach dem ersten bzw. zweiten Drittel des Reaktionsrohres seitlich angebracht sind. Im Hohlraum zwischen Nickelrohr (1) und Stahlrohr (2), der vertikal in drei gleichlange Segmente aufgeteilt ist, wird thermostatisiertes Heizöl unterschiedlicher Temperatur in drei Heizölkreisläufen I bis III umgepumpt. Die drei Heiz- und Kühlkreisläufe I bis III sind über die Reglerelemente (7, 8, 9) jeweils separat temperaturregelbar. Im oberen Heizkreislauf I beträgt die Regeltemperatur 215° C, im mittleren Heizkreislauf II 225° C und im unteren Heizkreislauf III 230° C. Das Reaktionsrohr (1) wird nach folgendem Füllplan mit Trägerkatalysatoren (von oben nach unten betrachtet) beschickt:

     13 cm Berlsättel mit 4 mm Durchmesser
     35 cm $\gamma$-Al$_2$O$_3$-Träger mit 7 % CuCl$_2$ und 3 % KCl
     23 cm $\gamma$-Al$_2$O$_3$-Träger mit 20 % CuCl$_2$ und 1,8 % KCl
     35 cm $\gamma$-Al$_2$O$_3$-Träger mit 10 % CuCl$_2$ und 3 % KCl
     23 cm $\gamma$-Al$_2$O$_3$-Träger mit 20 % CuCl$_2$ und 1,8 % KCl
     58 cm $\gamma$-Al$_2$O$_3$-Träger mit 20 % CuCl$_2$ und 1,8 % KCl
     13 cm Berlsättel mit 4 mm Durchmesser

Die einzelnen Gasströme werden über geeichte Rotameter zugeführt. 100 Nl/h Chlorwasserstoff und 58 Nl/h Ethylen werden zunächst vermischt und dann zusammen mit 57 Nl/h Luft über die Zuleitstelle (3) am Reaktoroberteil aufgegeben. Weitere 57 Nl/h Luft bzw. 30 Nl/h Luft werden über die Zuleitung (4) bzw. (5) zugegeben. Das den Reaktor über Leitung (10) verlassende Reaktionsgemisch wird im Intensivkühler (11) mit Wasser gekühlt, wobei Partialkondensation eintritt. Im Abscheider (12) wird die flüssige Phase, bestehend aus 1,2-Dichlorethan und Reaktionswasser, in dem nicht umgesetzter Chlorwasserstoff größtenteils gelöst ist, abgetrennt. Der nicht kondensierbare Gasstrom wird in der Kältefalle (13) auf -25° C abgekühlt, wobei weitere Kondensation eintritt und dann im anschließenden Wasserwäscher (14) Chlorwasserstoff-frei gewaschen. Die beiden Kondensate aus dem Abscheider (12) und der Kältefalle (13) werden vereint und nach Abtrennen der wäßrigen Phase durch Dekantieren, gaschromatographisch analysiert. Das Abgas nach der Kältefalle (13) wird nach Probenahme über die Gasmaus (15) gaschromatographisch auf Kohlenmonoxid und Kohlendioxid untersucht. Der Chlorwasserstoffumsatz wird aus dem Chlorwasserstoffgehalt in der vereinten wäßrigen Phase und im Ablauf des Wasserwäschers (14) errechnet. Die Reaktion wird bei Atmosphärendruck durchgeführt.

Zur Herstellung der erfindungsgemäßen Trägerkatalysatoren werden $\gamma$-Al$_2$O$_3$-Hohlextrudate mit folgenden Daten verwendet:

| | |
|---|---|
| spez. Gesamtoberfläche (BET): | 198 m$^2$/g |
| Gesamtporenvolumen: | 0,86 ml/g |
| Porenverteilung: | Keine Poren < 4 nm Durchmesser, 83 % der Poren mit einem Durchmesser von 8 - 20 nm, Rest Makroporen >20 nm; |
| Außendurchmesser: | 4,5 mm |
| Innendurchmesser: | 1,5 mm |
| Länge: | 11 ± 2 mm |

Die Katalysatoren werden wie folgt hergestellt:

a) 7 Gew.-% CuCl$_2$, 3 Gew.-% KCl auf $\gamma$-Al$_2$O$_3$-Hohlextrudaten als Trägerkörper
270 g Trägerkörper werden mit einer wäßrigen Lösung, welche 27 g CuCl$_2$ x 2H$_2$O und 9 g KCl enthält, imprägniert. Die Trocknung erfolgt bei 130° C. Der fertige Katalysator hat eine BET-Oberfläche von 169 m$^2$/g und ein Gesamtporenvolumen von 0,78 ml/g.

b) 10 Gew.-% CuCl$_2$, 3 Gew.-% KCl auf $\gamma$-Al$_2$O$_3$-Hohlextrudaten als Trägerkörper

261 g Trägerkörper werden mit einer wäßrigen Lösung, welche 38,5 g $CuCl_2$ x $2H_2O$ und 9 g KCl enthält, imprägniert. Die Trocknung erfolgt bei 130° C. Der fertige Katalysator hat eine BET-Oberfläche von 154 $m^2$/g und ein Gesamtporenvolumen von 0,72 ml/g.

c) 20 Gew.-% $CuCl_2$, 1,8 Gew.-% KCl auf $\gamma$-$Al_2O_3$-Hohlextrudaten als Trägerkörper

469 g Trägerkörper werden mit einer wäßrigen Lösung, welche 154 g $CuCl_2$ x $2H_2O$ und 11 g KCl enthält, imprägniert. Die Trocknung erfolgt bei 130° C. Der fertige Katalysator hat eine BET-Oberfläche von 126 $m^2$/g und ein Gesamtporenvolumen von 0,65 ml/g.

Die Prüfergebnisse sind nachfolgend in Tabelle 1 zusammengefaßt.

Vergleichsbeispiel 1a

Zur Herstellung der Trägerkatalysatoren werden $\gamma$-$Al_2O_3$-Hohlextrudate mit folgenden Daten verwendet:

| | |
|---|---|
| spez. Gesamtoberfläche (BET): | 242 $m^2$/g |
| Gesamtporenvolumen: | 0,50 ml/g |
| Porenverteilung: | Keine Poren < 2 nm Durchmesser, 75 % der Poren mit einem Durchmesser von 3 - 8 nm, Rest > 8 nm Durchmesser; |
| Außendurchmesser: | 4,5 mm |
| Innendurchmesser: | 1,5 mm |
| Länge: | 5 ± 1 mm |

Die Katalysatoren werden analog Beispiel 1 hergestellt.

| Katalysator | | BET-Oberfl. | Porenvol. |
|---|---|---|---|
| 7 Gew.-% $CuCl_2$, | 3 Gew.-% KCl | 197 $m^2$/g | 0,43 ml/g |
| 10 Gew.-% $CuCl_2$, | 3 Gew.-% KCl | 168 $m^2$/g | 0,38 ml/g |
| 20 Gew.-% $CuCl_2$, | 1,8 Gew.-% KCl | 110 $m^2$/g | 0,29 ml/g |

Die Ergebnisse sind in Tabelle 1 zusammengefaßt.

Vergleichsbeispiel 1 b

Zur Herstellung der Trägerkatalysatoren werden $\gamma$-$Al_2O_3$-Kugeln (4 - 6 mm Durchmesser) mit folgenden Daten verwendet:

| | |
|---|---|
| spez. Gesamtoberfläche (BET): | 222 $m^2$/g |
| Gesamtporenvolumen: | 0,60 ml/g |
| Porenverteilung: | Keine Poren < 2 nm Durchmesser, 60 % der Poren mit einem Durchmesser 2 - 8 nm, Rest > 8 nm; |

Die Katalysatoren werden analog Beispiel 1 hergestellt.

| Katalysator | BET-Oberfl. | Porenvol. |
|---|---|---|
| 7 Gew.-% $CuCl_2$, 3 Gew.-% KCl | 195 $m^2$/g | 0,54 ml/g |
| 10 Gew.-% $CuCl_2$, 3 Gew.-% KCl | 179 $m^2$/g | 0,49 ml/g |
| 20 Gew.-% $CuCl_2$, 1,8 Gew.-% KCl | 130 $m^2$/g | 0,41 ml/g |

Die Ergebnisse sind in Tabelle 1 zusammengefaßt.

Tabelle 1

| | Beispiel 1 | Vergleichs-beispiel 1 a | Vergleichs-beispiel 1 b |
|---|---|---|---|
| organisches Kondensat | 162,4 cm³/h | 153,2 cm³/h | 149,1 cm³/h |
| wäßriges Kondensat | 47,6 cm³/h | 38,5 cm³/h | 36,8 cm³/h |
| Abgasmenge | 135,4 Nl/h | 139,5 Nl/h | 145,9 Nl/h |
| HCl-Umsatz | 91,9 % | 88,7 % | 85,2 % |
| **Abgasanalyse** | | | |
| CO-Gehalt | 0,80 Vol% | 0,98 Vol% | 1,71 Vol% |
| CO$_2$-Gehalt | 0,62 Vol% | 1,25 Vol% | 1,55 Vol% |
| **Analyse des org. Kondensats** | | | |
| Ethylchlorid | 510 Gewppm | 1600 Gewppm | 1050 Gewppm |
| trans-1,2-Dichlorethylen | 180 Gewppm | 300 Gewppm | 500 Gewppm |
| 1,1-Dichlorethan | 150 Gewppm | 130 Gewppm | 180 Gewppm |
| Tetrachlorkohlenstoff | 2290 Gewppm | 2300 Gewppm | 3500 Gewppm |
| cis-1,2-Dichlorethylen | 560 Gewppm | 980 Gewppm | 1490 Gewppm |
| Chloroform | 1090 Gewppm | 1890 Gewppm | 2540 Gewppm |
| 1,2-Dichlorethan | 97,88 Gew.-% | 96,40 Gew.-% | 95,41 Gew.-% |
| Chloral | 1540 Gewppm | 2100 Gewppm | 2200 Gewppm |
| 1,1,2-Trichlorethan | 1,485 Gew.-% | 2,65 Gew.-% | 3,15 Gew.-% |

Die Gegenüberstellung in Tabelle 1 zeigt den großen technischen Fortschritt hinsichtlich Aktivität, Produktselektivität und Verbrennungsrate bei Verwendung des erfindungsgemäßen Trägerkatalysator im Vergleich zu herkömmlichen Katalysatoren, bei denen erwartungsgemäß die kugelförmigen Katalysatoren aus bekannten Gründen ringförmigen Katalysatoren unterlegen sind.

Beispiel 2

Es wurde eine Reaktoranlage verwendet, die aus drei in Reihe geschalteten gleichgroßen Rohrbündelreaktoren bestand. Jeder Reaktor enthielt 3 200 Nickelrohre mit einer lichten Weite von 27,5 mm. In den Nickelrohren befand sich der Katalysator gemäß Beispiel 1, der ein in Strömungsrichtung ansteigendes Aktivitätsprofil aufwies. Als Trägermaterial wurde $\gamma$-Al$_2$O$_3$ verwendet, das in Form von Ringen mit folgenden Abmessungen vorlag:

Außendurchmesser: 4,5 mm
Innendurchmesser: 1,5 mm

Länge: 11 ± 2 mm

Das in Strömungsrichtung ansteigende Aktivitätsprofil wurde dadurch verwirklicht, daß im Eingangsdrittel jedes Reaktors die Konzentration an Kupfer-II-Chlorid 7 Gew.-%, die Konzentration an Kaliumchlorid 3 Gew.-%, im mittleren Drittel jeweils 10 Gew.-% $CuCl_2$ und 3 Gew.-% KCl und im letzten Drittel jeweils etwa 20 Gew.-% $CuCl_2$ und 1,8 Gew.-% KCl betrug. Das Katalysatorvolumen betrug pro Reaktor 6,8 m³. Das Gesamtporenvolumen des Trägerkatalysators lag je nach Aktivkomponentenbeladung bei Werten von 0,78, 0,72 und 0,65 ml/g, wobei etwa 83 % des Porenvolumens durch Poren mit einem Durchmesser von 8 bis 20 Nanometer gebildet wurden.

Die spezifische Oberfläche des Trägerkatalysators lag je nach Beladung mit den Aktivkomponenten bei Werten von 169, 154 und 126 m²/g. Die Nickelrohre waren mit einem Außenmantel umgeben, in dem sich als Kühlmedium zur Abführung der freiwerdenden Reaktionswärme Wasser befand, das unter einem Druck von 19 bar abs. im ersten Reaktor, 21 bar abs. im zweiten Reaktor und 24 bar abs. im dritten Reaktor stand.

Als Sauerstoffquelle wurde Luft verwendet, die durch Zugabe von reinem Sauerstoff auf einen Sauerstoffgehalt von 30 Vol% gebracht wurde. In den ersten Reaktor wurde bei Reglerdrücken von 8 bar abs. ein Gemisch aus 437,5 kmol/h Chlorwasserstoff, 220 kmol/h Ethylen und 160 kmol/h auf 30 Vol% Sauerstoff angereicherter Luft eingeschleust. Die mit Sauerstoff angereicherte Luft wurde noch vor Vermischung mit den anderen Reaktanden nach Vorwärmen auf 180° C mit 48 kmol/h Wasserdampf beaufschlagt. Die Temperatur des Reaktionsgemisches vor Eintritt in den ersten Reaktor betrug 135° C.

Das den ersten Reaktor verlassende Reaktionsgemisch wurde vor Eintritt in den zweiten Reaktor mit 172 kmol/h auf 30 Vol% Sauerstoff angereicherte Luft beaufschlagt. Schließlich wurde dem Reaktionsgemisch zwischen zweiten und dritten Reaktor noch 70 kmol/h auf 30 Vol% Sauerstoff angereicherte Luft zugeführt. Der Systemdruck vor dem ersten Reaktor betrug 6,4 bar abs. Der Druckabfall über den Reaktoren betrug 0,37 bar im ersten Reaktor, 0,50 bar im zweiten Reaktor und 0,54 bar im dritten Reaktor. Die Hot Spot Temperaturen lagen im ersten Reaktor bei 289° C, im zweiten bei 280° C und im dritten Reaktor bei 235° C.

Das mit einer mittleren Temperatur von 220° C den dritten Reaktor verlassende Reaktionsgemisch wurde gekühlt und kondensiert. Das flüssige Kondensat wurde in einem nachgeschalteten Dekanter in eine organische und eine wäßrige Phase getrennt. Die organische Phase hatte folgende Zusammensetzung:

| | |
|---|---|
| Ethylchlorid | 0,22 Gew.-% |
| 1,2-Dichlorethan | 0,01 Gew.-% |
| Chloroform | 0,04 Gew.-% |
| Tetrachlorkohlenstoff | 0,07 Gew.-% |
| Dichlorethylene | 0,01 Gew.-% |
| Chloral | 0,20 Gew.-% |
| 1,1,2-Trichlorethan | 0,45 Gew.-% |
| Sonstige | 0,10 Gew.-% |
| 1,2-Dichlorethan | 98,90 Gew.-% |

Die Analyse der kondensierten wäßrigen Phase ergab:

| | |
|---|---|
| Chlorwasserstoff | 1,94 Gew.-% |
| Chloralhydrat | 0,38 Gew.-% |
| 1,2-Dichlorethan | 0,58 Gew.-% |

Damit errechnet sich aus der Chlorwasserstoffkonzentration ein Chlorwasserstoffumsatz von 99,4 %.

Die Analyse des nicht kondensierbaren Abgasstromes (im wesentlichen Stickstoff aus der Luft) ergab folgende Konzentration an Ethylenverbrennungsprodukten:

| | |
|---|---|
| Kohlenmonoxid: | 1,14 Vol% |
| Kohlendioxid: | 0,96 Vol% |

Die Produktionsrate betrug 21 500 kg 1,2-Dichlorethan pro Stunde. Die Raum-Zeit-Leistung lag somit bei 1 054 kg 1,2-Dichlorethan pro m³ Katalysator x Stunde, bezogen auf alle 3 Reaktoren.

Es wurde, bezogen auf alle 3 Reaktoren, eine durchschnittliche spezifische Produktausbringung von 17 850 to 1,2-Dichlorethan pro m³ Katalysator erzielt, wobei der Katalysator im ersten Reaktor eine Laufzeit von 8 400 Stunden, im zweiten Reaktor von 24 800 Stunden und im dritten Reaktor von 17 600 Stunden erreichte.

Vergleichsbeispiel 2

Die Arbeitsweise gemäß Beispiel 2 wurde wiederholt mit dem Katalysator aus Vergleichsbeispiel 1 a:

| | |
|---|---|
| Außendurchmesser der Ringe: | 4,5 mm |
| Innendurchmesser der Ringe: | 1,5 mm |
| Länge der Ringe: | 5 ± 1 mm |

Die spezifische Oberfläche betrug je nach Konzentration an Aktivkomponenten 197, 168 und 110 m²/g,

7

das Gesamtporenvolumen lag bei 0,43, 0,38 und 0,29 ml/g, wobei 75 % der Poren einen Bereich von 3 bis 8 Nanometer Durchmesser aufwiesen.

Die Konzentrationen an Aktivkomponenten sowie der Füllplan waren analog dem Beispiel 2. Aufgrund eines höheren Druckabfalls über den Reaktoren mußte der Durchsatz der Anlage insgesamt um 15 % zurückgenommen werden, wobei dies gleichmäßig, bezogen auf die Einzelbeaufschlagung der 3 Reaktoren, gemäß Beispiel 2 erfolgte.

Der Systemdruck vor dem ersten Reaktor stellte sich bei gleichem Druck am Reaktionsende wie in Beispiel 2 trotzdem auf den gleichen Wert von 6,4 bar abs. ein, wodurch der Gesamtdurchsatz nach oben begrenzt war. Die Druckverluste und die Temperaturen in den einzelnen Reaktoren waren:

im ersten Reaktor 0,57 bar        Hot Spot 298° C  
im zweiten Reaktor 0,64 bar       Hot Spot 291° C  
im dritten Reaktor 0,70 bar       Hot Spot 245° C  

Die einzelnen Produkt- bzw. Abgasströme hatten folgende Zusammensetzung:

Wäßrige Phase: 4,8 Gew.-% Chlorwasserstoff entsprechend einem Chlorwasserstoffumsatz von etwa 98,5 Gew.-%.

Organische Phase:

| | |
|---|---|
| Ethylchlorid | 0,65 Gew.- |
| 1,1-Dichlorethan | 0,01 Gew.-% |
| Chloroform | 0,08 Gew.-% |
| Tetrachlorkohlenstoff | 0,12 Gew.-% |
| Dichlorethylen | 0,02 Gew.-% |
| Chloral | 0,35 Gew.-% |
| 1,1,2-Trichlorethan | 0,65 Gew.-% |
| Sonstige | 0,15 Gew.-% |
| 1,2-Dichlorethan | 97,97 Gew.-% |

| | | |
|---|---|---|
| __Abgas__ | Kohlenmonoxid: | 1,83 Vol% |
| | Kohlendioxid: | 1,55 Vol% |

Die Produktionsrate betrug 18 275 kg 1,2-Dichlorethan pro Stunde. Die Raum-Zeit-Leistung lag somit bei 896 kg 1,2-Dichlorethan pro $m^3$ Katalysator x Stunde, bezogen auf alle 3 Reaktoren. Es wurde, bezogen auf alle 3 Reaktoren, eine durchschnittliche spezifische Produktausbringung von 15 844 to 1,2-Dichlorethan pro $m^3$ Katalysator erzielt, wobei trotz insgesamt geringem Durchsatz der Katalysator im ersten Reaktor eine Laufzeit von nur 9 100 Stunden, im zweiten Reaktor von 25 600 Stunden und im dritten Reaktor von 18 300 Stunden erreichte.

Die Ergebnisse von Beispiel 2 verdeutlichen den technischen Fortschritt des erfindungsgemäßen Katalysators gegenüber herkömmlichen Katalysatoren hinsichtlich Aktivität, Produktselektivität, Verbrennungsrate und damit Ehtylenausbeute, Druckabfall und damit Raum-Zeit-Leistung bzw. spezifischer Produktausbringung pro $m^3$ Katalysator.

Beispiel 3

Es wurde der in Beispiel 1 beschriebene und in Fig. 1 gezeigte Laborreaktor verwendet. Desgleichen kam der gleiche Katalysator und derselbe Füllplan wie in Beispiel 1 zur Anwendung. Die Reaktionsbedingungen waren ebenfalls analog Beispiel 1 mit der Ausnahme, daß anstelle von Luft als Sauerstoffquelle reiner Sauerstoff verwendet wurde.

Um die Kreisgasfahrweise im ethylenreichen Bereich gemäß US-PS 3 892 816 aufgrund eines fehlenden Kreisgaskompressors annähernd zu simulieren, wurden folgende Mengen an Reaktanden eingesetzt:

100 Nl/h Chlorwasserstoff, vermischt mit 390 Nl/h Ethylen und  
10,5 Nl/h Sauerstoff über Zuleitstelle (3),

10,5 Nl/h Sauerstoff über Zuleitstelle (4),

5,25 Nl/h Sauerstoff über Zuleitstelle (5).

Die Aufarbeitung und analytische Auswertung des Reaktionsproduktes erfolgte analog Beispiel 1.

Die Ergebnisse sind in Tabelle 2 zusammengefaßt.

Vergleichsbeispiel 3

Es wurde analog Beispiel 3 verfahren, jedoch der Katalysator von Vergleichsbeispiel 1 a verwendet. Die Ergebnisse sind in Tabelle 2 festgehalten.

Die Gegenüberstellung der Ergebnisse der Sauerstoff-Fahrweise im sog. fetten Bereich zeigt, daß der erfindungsgemäße Katalysator auch bei reiner Sauerstoff-Fahrweise einen großen technischen Fortschritt besonders hinsichtlich Produktselektivität und Verbrennungsrate bringt. Naturgemäß ist die Produktselektivität höher bzw. die Verbrennungsrate niedriger, wenn anstelle von Luft reiner Sauerstoff und ein extrem hoher Ethylenüberschuß zur Anwendung kommen, da Ethylen gegenüber Stickstoff (aus der Luft) aufgrund seiner weitaus höheren spezifischen Wärmekapazität die Wärmeabführung stark begünstigt.

Durch den bei Sauerstoff-Fahrweise benötigten extrem hohen Ethylenüberschuß, bezogen auf die Stöchiometrie zu Chlorwasserstoff, ist zwangsläufig auch der Chlorwasserstoffumsatz höher als bei Luft-Fahrweise.

Tabelle 2

| | Beispiel 3 | Vergleichs-beispiel 3 |
|---|---|---|
| organisches Kondensat | 169,4 cm³/h | 169,0 cm |
| wäßriges Kondensat | 50,4 cm³/h | 50,5 cm³/h |
| Abgasmenge | 357 Nl/h | 355 Nl/h |
| HCl-Umsatz | 99,8 % | 99,7 % |
| Abgasanalyse | | |
| CO-Gehalt | 0,15 Vol% | 0,36 Vol% |
| CO₂-Gehalt | 0,30 Vol% | 0,63 Vol% |
| Analyse des org. Kondensates | | |
| Ethylchlorid | 390 Gewppm | 1320 Gewppm |
| trans-1,2-Dichlorethylen | < 1 Gewppm | < 1 Gewppm |
| 1,1-Dichlorethan | 100 Gewppm | 180 Gewppm |
| Tetrachlorkohlenstoff | 930 Gewppm | 1490 Gewppm |
| cis-1,2-Dichlorethylen | < 1 Gewppm | < 1 Gewppm |
| Chloroform | 690 Gewppm | 1080 Gewppm |
| 1,2-Dichlorethan | 99,68 Gew.-% | 99,39 Gew.-% |
| Chloral | 640 Gewppm | 960 Gewppm |
| 1,1,2-Trichlorethan | 410 Gewppm | 1080 Gewppm |

**Patentansprüche**

1. Zylindrisch geformter Trägerkatalysator, enthaltend Kupferionen und Alkaliionen auf einem ringförmigen Träger aus oberflächenaktivem Material,
   **dadurch gekennzeichnet,**
   daß
   a) das ringförmige Trägermaterial einen Außendurchmesser von 4 bis 6 mm, einen Innendurchmesser von 1 bis 2 mm und eine Höhe aufweist, die das 1,7 bis 3,75 fache, vorzugsweise das 2,0 bis 3,00 fache des Außendurchmessers ausmacht,
   b) der die Kupferionen und Alkaliionen enthaltende Trägerkatalysator ein Gesamtporenvolumen von 0,6 bis 1,0 ml/g hat und
   c) Poren < 4 Nanometer Porendurchmesser nicht vorhanden sind, mindestens 80 % des Gesamtporenvolumens durch Poren mit einem Durchmesser von 8 bis 20 Nanometer gebildet werden und der Rest aus Makroporen größer 20 Nanometer besteht.

2. Trägerkatalysator nach Anspruch 1,
   **dadurch gekennzeichnet,**
   daß der Träger aus Gamma-Aluminiumoxid mit einer spezifischen Oberfläche von 120 bis 215 m²/g und einem Porenvolumen von 0,6 bis 1,0 ml/g besteht.

3. Verwendung des Trägerkatalysators nach Anspruch 1 oder 2 zur Herstellung von 1,2-Dichlorethan durch Oxichlorierung von Ethylen mit Luft oder mit Sauerstoff angereicherter Luft oder mit reinem Sauerstoff unter Kreisgasführung des überschüssigen, nicht umgesetzten Ethylens.

## Claims

1. A cylidrically shaped supported catalyst containing copper ions and alkali metal ions on an annular support of surface-active material, characterized in that
   a) the annular support material has an external diameter of 4 to 6 mm, an internal diameter of 1 to 2 mm and a height of 1.7 to 3.75 times and preferably 2.0 to 3.00 times its external diameter,
   b) the supported catalyst containing the copper ions and alkali metal ions has a total pore volume of 0.6 to 1.0 ml/g and
   c) there are no pores with a pore diameter of < 4 nanometers and at least 80% of the total pore volume is made up of pores with a diameter of 8 to 20 nanometers, the rest being made up of macropores larger than 20 nanometers in diameter.

2. A supported catalyst as claimed in claim 1, characterized in that the support consists of $\gamma$-aluminium oxide with a specific surface of 120 to 215 m²/g and a pore volume of 0.6 to 1.0 ml/g.

3. The use of the supported catalyst claimed in claim 1 or 2 for the production of 1,2-dichloroethane by oxychlorination of ethylene with air or with oxygen-enriched air or with pure oxygen, excess unreacted ethylene being recirculated.

## Revendications

1. Catalyseur fixé sur support, de forme cylindrique, contenant des ions cuivre et des ions de métal alcalin, sur un support annulaire constitué d'un matériau à surface active, caractérisé en ce que
   a) le matériau de support annulaire a un diamètre externe de 4 à 6 mm, un diamètre interne de 1 à 2 mm et une hauteur qui représente de 1,7 à 3,75 fois, de préférence de 2,0 à 3,00 fois, le diamètre externe.
   b) le catalyseur fixé sur support, contenant des ions cuivre et des ions de métal alcalin, a un volume total de pores de 0,6 à 1,0 ml/g, et
   c) des pores à diamètre de pore < 4 nm ne sont pas présents, au moins 80 % du volume total de pores sont constitués par des pores ayant un diamètre de 8 à 20 nm, et le reste est constitué de macropores de plus de 20 nm.

2. Catalyseur fixé sur support selon la revendication 1, caractérisé en ce que le support est constitué d'oxyde d'aluminium gamma ayant une surface spécifique de 120 à 215 m²/g et un volume de pores de 0,6 à 1,0 ml/g.

3. Utilisation du catalyseur fixé sur support, selon la revendication 1 ou 2, pour la production du 1,2-dichloréthane par oxychloration de l'éthylène à l'air ou avec de l'air enrichi en oxygène ou avec de l'oxygène pur, avec recyclage sous forme de gaz, de l'éthylène en excès n'ayant pas réagi.

*Fig. 1*